(19) Europäisches Patentamt
European Patent Office
Office européen des brevets

(11) Veröffentlichungsnummer: **0 387 603 A1**

(12) # EUROPÄISCHE PATENTANMELDUNG

(21) Anmeldenummer: **90103842.2**

(22) Anmeldetag: **28.02.90**

(51) Int. Cl.[5]: **C07D 401/12, C07D 401/06, C07D 409/14, A61K 31/44**

(30) Priorität: **11.03.89 DE 3907974**

(43) Veröffentlichungstag der Anmeldung: **19.09.90 Patentblatt 90/38**

(84) Benannte Vertragsstaaten: **AT BE CH DE ES FR GB IT LI NL SE**

(71) Anmelder: **MERCK PATENT GESELLSCHAFT MIT BESCHRÄNKTER HAFTUNG**
**Frankfurter Strasse 250 Postfach 4119**
**D-6100 Darmstadt(DE)**

(72) Erfinder: **Böttcher, Henning, Dr.**
**Soderstrasse 95**
**D-6100 Darmstadt(DE)**
Erfinder: **Juraszyk, Horst, Dr.**
**Kleiner Ring 24**
**D-6104 Seeheim(DE)**
Erfinder: **Hausberg, Hans-Heinrich, Dr.**
**Wittenberger Strasse 12**
**D-6105 Ober-Ramstadt(DE)**
Erfinder: **Greiner, Hartmut, Dr.**
**Dieburgerstrasse 218**
**D-6100 Darmstadt(DE)**
Erfinder: **Seyfried, Christoph, Dr.**
**Mathildenstrasse 6**
**D-6104 Jugenheim(DE)**
Erfinder: **Minck, Klaus-Otto, Dr.**
**Büchestrasse 8**
**D-6105 Ober-Ramstadt(DE)**
Erfinder: **Bergmann, Rolf, Dr.**
**Birkenhag 36**
**D-6101 Reichelsheim(DE)**

(54) **Indolderivate.**

(57) Indolderivate der Formel I

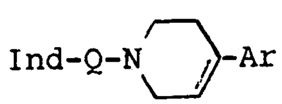

I

worin Ind, Q und Ar die in Patentanspruch 1 angegebenen Bedeutungen haben, und deren Salze zeigen Wirkungen auf das Zentralnervensystem.

EP 0 387 603 A1

# Indolderivate

Die Erfindung betrifft neue Indolderivate der Formel I

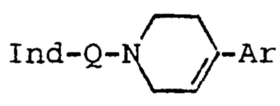

I

worin

Ind einen durch $-O-CH_2-CO-R^1$, $-NHR^2$, $-NO_2$, $-CO-NR^3R^4$ oder $-CSNH_2$ substituierten Indol-3-ylrest, $R^1$ OH, OA, $NH_2$, NHA, $NA_2$, $NH-CH_2COOA$ oder $NHCH(CH_2OH)COOA$,

$R^2$ H, Ac, $CONH_2$, CONHA, $CONA_2$ oder $SO_2A$,

$R^3$ H, A oder Hydroxyalkyl,

$R^4$ Hydroxyalkyl, AO-alkyl, AcO-alkyl, ANH-CO-O-alkyl, AOOC-alkyl, $H_2NCO$-alkyl, $HSO_3$-alkyl, $A_2N$-alkyl, Ar, Ar-alkyl oder Het-alkyl,

$R^3$ und $R^4$ zusammen auch eine Alkylengruppe mit 3-7 C-Atomen, die durch O oder $NR^5$ unterbrochen und/oder durch $NA_2$, NHAc, COOA, $CONH_2$, Ar oder Het substituiert sein und/oder eine zusätzliche Doppelbindung enthalten kann,

$R^5$ H, A, Ar, Het, Ac, COOA, $CH_2CONH_2$ $CH_2CONHA$, $CH_2CONA_2$ oder $CH_2CONR^6$,

$R^6$ Alkylen mit 3-7 C-Atomen,

Q $-(CH_2)_n-$, $-CH_2-S-CH_2CH_2-$, $-CH_2-SO-CH_2CH_2-$ oder $-CH_2-SO_2-CH_2CH_2-$,

n 2, 3, 4 oder 5,

A Alkyl mit 1-4 C-Atomen,

-alkyl- Alkylen mit 1-4 C-Atomen,

Ar eine unsubstituierte oder eine ein- oder zweifach durch F, Cl, Br, OA und/oder OH oder durch eine Methylendioxygruppe substituierte Phenylgruppe oder eine 2- oder 3-Thienylgruppe,

Ac A-CO- oder Ar-CO- und

Het einen gesättigten oder ungesättigten 5- oder 6-gliedrigen heterocyclischen Rest mit 1-4 N-, O- und/oder S-Atomen, der mit einem Benzolring kondensiert und/oder ein- oder zweifach durch A substituiert sein kann,

bedeuten,

sowie deren Salze,

Der Erfindung lag die Aufgabe zugrunde, neue Verbindungen aufzufinden, die zur Herstellung von Arzneimitteln verwendet werden können.

Es wurde gefunden, daß die Verbindungen der Formel I und ihre physiologisch unbedenklichen Säureadditionssalze wertvolle pharmakologische Eigenschaften besitzen. So zeigen sie insbesondere Wirkungen auf das Zentralnervensystem, vor allem Dopamin-stimulierende (Anti-Parkinson) sowie serotoninagonistische und -antagonistische Wirkungen. Im einzelnen induzieren die Verbindungen der Formel I contralaterales Drehverhalten in Hemiparkinson-Ratten (feststellbar nach der Methode von Ungerstedt et al., Brain Res. 24 (1970), 485-493). Sie hemmen die Bindung von tritiierten Dopaminagonisten und -antagonisten an striäre Rezeptoren (feststellbar nach der Methode von Schwarcz et al., J. Neurochemistry 34 (1980), 772-778 und Creese et al., European J. Pharmacol. 46 (1977), 377-381) und die Bindung von tritiierten Serotoninliganden an hippocampale Rezeptoren (Cossery et al., European J. Pharmacol 140 - (1987), 143-155). Außerdem treten Veränderungen der DOPA-Akkumulation im Striatum und der 5-HTP-Akkumulation im N.raphe auf (Seyfried et al., European J. Pharmacol. 160 (1989), 31-41). Zusätzlich hemmen die Verbindungen den Zungen-Kieferreflex bei der narkotisierten Ratte (feststellbar in Anlehnung an die Methoden von Barnett et al., European J. Pharmacol. 21 (1973), 178-182, und von Ilhan et al., European J. Pharmacol. 33 (1975), 61-64). Weiterhin treten analgetische und blutdrucksenkende Wirkungen auf; so wird der bei kathetertragenden wachen, spontan hypertonen Ratten (Stamm SHR/Okamoto/NIH-MO-CHB-Kisslegg; Methode vgl. Weeks und Jones, Proc. Soc. Exptl. Biol. Med. 104 (1960), 646-648) der direkt gemessene Blutdruck nach peroraler Gabe der Verbindungen gesenkt.

Verbindungen der Formel I und ihre physiologisch unbedenklichen Säureadditionssalze können daher als Arzneimittelwirkstoffe für Anxiolytika, Antidepressiva, Neuroleptika, Anti-Parkinson-Mittel und/oder Antihypertonika und auch als Zwischenprodukte zur Herstellung anderer Arzneimittelwirkstoffe verwendet werden.

Gegenstand der Erfindung sind die Indolderivate der Formel I sowie ihre physiologisch unbedenklichen

Säureadditionssalze.

Der Rest A bedeutet Alkyl mit 1, 2, 3 oder 4 insbesondere 1 oder 2 C-Atomen, vorzugsweise Methyl, ferner auch Ethyl, n-Propyl, Isopropyl, n-Butyl, Isobutyl, sek.-Butyl oder tert.-Butyl. OA ist vorzugsweise Methoxy, ferner auch Ethoxy, n-Propoxy, Isopropoxy, n-Butoxy, Isobutoxy, sek.-Butoxy oder tert.-Butoxy. NHA ist vorzugsweise Methylamino, ferner Ethylamino, n-Propylamino, Isopropylamino, n-Butylamino, Isobutylamino, sek.-Butylamino oder tert.-Butylamino. $NA_2$ bedeutet vorzugsweise Dimethylamino, ferner N-Ethyl-N-methyl-amino, Diethylamino, Di-n-propylamino, Diisopropylamino oder Di-n-butylamino.

Analog bedeutet NH-$CH_2$COOA vorzugsweise Methoxycarbonylmethyl-amino oder Ethoxycarbonylmethyl-amino; NHCH($CH_2$OH)COOA vorzugsweise 1-Methoxycarbonyl-2-hydroxy-ethylamino oder 1-Ethoxycarbonyl-2-hydroxy-ethylamino; CONHA vorzugsweise N-Methyl-carbamoyl oder N-Ethyl-carbamoyl; $CONA_2$ vorzugsweise N,N-Dimethyl-carbamoyl oder N,N-Diethyl-carbamoyl; CS-NH-COOA vorzugsweise Methoxycarbonylamino-thioxo oder Ethoxycarbonylamino-thioxo; $SO_2A$ vorzugsweise Methylsulfonyl oder Ethylsulfonyl; COOA vorzugsweise Methoxycarbonyl oder Ethoxycarbonyl; $CH_2$CONHA vorzugsweise N-Methylcarbamoyl-methyl oder N-Ethylcarbamoyl-methyl; $CH_2CONA_2$ vorzugsweise N,N-Dimethyl-carbamoylmethyl oder N,N-Diethyl-carbamoyl-methyl.

Die Gruppe -alkyl- steht für eine geradkettige oder verzweigte Alkylengruppe mit insbesondere 1 oder 2 C-Atomen, vorzugsweise für $-CH_2-$ oder $-CH_2CH_2-$, aber auch z.B. für $-CH(CH_3)-$, $-(CH_2)_3-$, $-CH(CH_3)CH_2-$, $-CH_2CH(CH_3)-$, $-C(CH_3)_2-$, $-(CH_2)_4-$.

Dementsprechend bedeutet Hydroxyalkyl vorzugsweise Hydroxymethyl oder 1- oder 2-Hydroxyethyl, ferner bevorzugt 3-Hydroxypropyl, 4-Hydroxybutyl oder 2-Hydroxy-1,1-dimethylethyl; AO-alkyl vorzugsweise Methoxymethyl, Ethoxymethyl, 1- oder 2-Methoxyethyl, 1- oder 2-Ethoxyethyl; ANH-CO-O-alkyl vorzugsweise N-Methyl-carbamoyl-oxy-methyl, N-Ethyl-carbamoyl-oxy-methyl, 1- oder 2-(N-Methyl-carbamoyl-oxy)-ethyl, 1- oder 2-(N-Ethyl-carbamoyl-oxy)-ethyl; AOOC-alkyl, vorzugsweise Methoxycarbonylmethyl, Ethoxycarbonylmethyl, 1- oder 2-Methoxycarbonyl-ethyl, 1- oder 2-Ethoxycarbonyl-ethyl; $H_2N$-CO-alkyl vorzugsweise Carbamoylmethyl, 1- oder 2-Carbamoylethyl; $HSO_3$-alkyl vorzugsweise Sulfomethyl, 1- oder 2-Sulfoethyl; $A_2N$-alkyl vorzugsweise Dimethylaminomethyl, Diethylaminomethyl, 1- oder 2-Dimethylaminoethyl, 1- oder 2-Diethylaminoethyl.

Der Rest Ar ist bevorzugt unsubstituiertes Phenyl. Falls Ar eine substituierte Phenylgruppe bedeutet, so ist diese vorzugsweise einfach substituiert. Sie kann jedoch auch zweifach substituiert sein, wobei die Substituenten gleich oder verschieden sein können. Bevorzugte Substituenten an der Phenylgruppe sind F, Cl, Methoxy oder OH. Im einzelnen ist Ar bevorzugt Phenyl, o-, m- oder p-Fluorphenyl, o-, m-oder p-Chlorphenyl, o-, m- oder p-Methoxyphenyl, o-, m-oder p-Hydroxyphenyl, ferner o-, m- oder p-Bromphenyl, o-, m- oder p-Ethoxyphenyl, 2,3-, 2,4-, 2,5-, 2,6-, 3,4- oder 3,5-Dimethoxyphenyl, 3-Hydroxy-4-methoxyphenyl, 3-Methoxy-4-hydroxyphenyl, 2,3-, 2,4-, 2,5-, 2,6-, 3,4- oder 3,5-Dihydroxyphenyl, 2,3- oder 3,4-Methylendioxyphenyl, 2-oder 3-Thienyl.

Dementsprechend ist Ar-alkyl vorzugsweise Benzyl, 1- oder 2-Phenylethyl, o-, m- oder p-Fluorbenzyl, o-, m- oder p-Chlorbenzyl, o-, m- oder p-Methoxybenzyl, 1- oder 2-o-, -m- oder -p-Methoxyphenyl-ethyl, o-, m- oder p-Hydroxybenzyl, 1- oder 2-o-, -m- oder -p-Hydroxyphenyl-ethyl.

Ac ist vorzugsweise Acetyl oder Benzoyl, ferner bevorzugt Propionyl, Butyryl, Isobutyryl, o-, m- oder p-Fluorbenzoyl, o-, m- oder p-Chlorbenzoyl, o-, m- oder p-Hydroxybenzoyl, o-, m- oder p-Methoxybenzoyl. Dementsprechend ist AcO-alkyl vorzugsweise Acetoxymethyl, 1- oder 2-Acetoxy-ethyl, Benzoyloxymethyl, 1- oder 2-Benzoyloxyethyl; NHAc ist vorzugsweise Acetamido oder Benzamido.

Het ist vorzugsweise 2- oder 3-Furyl, 2- oder 3-Thienyl, 1-, 2- oder 3-Pyrrolyl, 1-, 2-, 4- oder 5-Imidazolyl, 1-, 3-, 4- oder 5-Pyrazolyl, 2-, 4- oder 5-Oxazolyl, 3-, 4-oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4-oder -5-yl, 1,2,4-Triazol-1-, -3- oder -5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 2-, 3-, 4-, 5- oder 6-2H-Thiopyranyl, 2-, 3- oder 4-4H-Thiopyranyl, 3- oder 4-Pyridazinyl, Pyrazinyl, 2-, 3-, 4-, 5-, 6- oder 7-Benzofuryl, 2-, 3-, 4-, 5-, 6- oder 7-Benzothienyl, 1-, 2-, 3-, 4-, 5-, 6- oder 7-Indolyl, 1-, 2-, 3-, 4-, 5-, 6- oder 7-Isoindolyl, 1-, 2-, 4-oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 1-, 2-, 3-, 4- oder 9-Carbazolyl, 1-, 2-, 3-, 4-, 5-, 6-, 7-, 8- oder 9-Acridinyl, 3-, 4-, 5-, 6-, 7- oder 8-Cinnolyl, 2-, 4-, 5-, 6-, 7- oder 8-Chinazolyl. Die heterocyclischen Reste können auch teilweise oder vollständig hydriert sein. Het kann also z.B. auch bedeuten 2,3-Dihydro-2-, -3-, -4- oder -5-furyl, 2,5-Dihydro-2-, -3-, -4- oder -5-furyl, Tetrahydro-2- oder -3-furyl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4-

oder -5-pyrazolyl, Tetrahydro-1-, -3-oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1,2,3,6-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-, -2-, -3-, -4-, -5-, -6-, -7- oder 8-isochinolyl.

Die heterocyclischen Reste können auch wie angegeben substituiert sein. Het kann z.B. bevorzugt auch bedeuten: 4- oder 5-Methyl-2-thiazolyl, 4-, 5- oder 6-Methyl-2-pyrimidinyl, 4,5-Dimethyl-2-thiazolyl, 3-, 4- oder 5-Methyl-2-furyl, 2-, 4- oder 5-Methyl-3-furyl, 2,4-Dimethyl-3-furyl, 3-, 4- oder 5-Methyl-2-thienyl, 2-, 4- oder 5-Methyl-3-thienyl, 3-Methyl-5-tert.-butyl-2-thienyl.

Der Rest Ind bedeutet einen einfach durch einen der angegebenen Reste substituierten Indol-3-ylrest. Vorzugsweise ist er in der 5-Stellung, ferner auch in der 4-, 6- oder 7-Stellung substituiert. Weiterhin ist eine Substitution in 1- oder 2-Stellung möglich. Bevorzugte Substituenten am Indol-3-ylrest sind $-NHR^2$, $-NO_2$ und $-CONR^3R^4$.

$R^1$ ist bevorzugt OH, $NH_2$, $NA_2$ $NHCH_2COOA$ oder $NHCH(CH_2OH)COOA$.

$R^2$ ist vorzugsweise $CONH_2$, ferner bevorzugt H, Ac oder $SO_2A$,

$R^3$ ist vorzugsweise H.

$R^4$ ist vorzugsweise Hydroxyalkyl, ferner bevorzugt AOOC-alkyl, $H_2NCO$-alkyl, $A_2N$-alkyl, Ar oder $HSO_3$-alkyl.

Die Gruppe $-NR^3R^4$ ist bevorzugt auch Pyrrolidino, Piperidino, Morpholino, 4-$R^5$-piperazino oder 4-$R^7$-piperidino, wobei $R^5$ vorzugsweise H, A, Ar, 2-Pyrimidinyl, 5-Methylthiazolyl, Ar-CO, COOA, $CH_2CONHA$ oder Pyrrolidinocarbonylmethyl und $R^7$ vorzugsweise $NA_2$, NHCOAr, COOA, $CONH_2$, Piperidino oder Morpholino, aber auch NHCOA, Ar oder eine andere Gruppe Het bedeuten.

Der Parameter n ist vorzugsweise 4, der Rest Q ist vorzugsweise $-(CH_2)_4-$ oder $-CH_2S-CH_2CH_2-$, weiterhin $-CH_2-SO-CH_2CH_2-$, $-CH_2-SO_2-CH_2CH_2-$, $-(CH_2)_2-$, $-(CH_2)_3-$ oder $-(CH_2)_5-$.

Dementsprechend sind Gegenstand der Erfindung insbesondere diejenigen Verbindungen der Formel I, in denen mindestens einer der genannten Reste eine der vorstehend angegebenen, insbesondere der vorstehend angegebenen bevorzugten Bedeutungen hat. Einige bevorzugte Gruppen von Verbindungen können durch die folgenden Teilformeln Ia bis If ausgedrückt werden, die der Formel I entsprechen und worin die nicht näher bezeichneten Reste und Parameter die bei Formel I angegebene Bedeutung haben, worin jedoch

in Ia Ind einen in 5-Stellung durch $-O-CH_2-CO-R^1$ substituierten Indol-3-yl-rest bedeutet;

in Ib Ind einen in 5-Stellung durch-$NHR^3$ substituierten Indol-3-yl-rest bedeutet;

in Ic Ind einen 5-Nitroindol-3-ylrest bedeutet;

in Id Ind einen in 5-Stellung durch $-CONR^3R^4$ substituierten Indol-3-ylrest bedeutet;

in Ie Ind einen in 5-Stellung durch $-CSNH_2$ substituierten Indol-3-yl-rest bedeutet;

in If Ind einen 5-Ureido-indol-3-yl-rest bedeutet.

Insbesondere sind bevorzugt Verbindungen der Teilformeln Ig sowie Iag bis Ifg, die den Teilformeln I sowie Ia bis If entsprechen, worin jedoch zusätzlich

Q $-(CH_2)_4-$ bedeutet.

Ferner sind insbesondere bevorzugt Verbindungen der Teilformeln Ih, Iah bis Ifh, Igh sowie Iagh bis Ifgh, die den Teilformeln I, Ia bis If, Ig sowie Iag bis Ifg entsprechen, worin jedoch zusätzlich

Ar Phenyl bedeutet.

Gegenstand der Erfindung ist ferner ein Verfahren zur Herstellung von Indolderivaten der Formel I sowie von deren Salzen, dadurch gekennzeichnet, daß man eine Verbindung der Formel II

$Ind-Q-X^1$ II

worin

$X^1$ X oder $NH_2$ und

X Cl, Br, J, OH oder eine reaktionsfähig funktionell abgewandelte OH-Gruppe bedeuten und

Ind und Q die angegebenen Bedeutungen haben,

mit einer Verbindung der Formel III

$X^2-CH_2CH_2CAr=CH-CH_2X^3$ III

worin

$X^2$ und $X^3$ gleich oder verschieden sein können und, falls $X^1 = NH_2$ ist, jeweils X, andernfalls zusammen NH bedeuten und

Ar die angegebene Bedeutung hat,

umsetzt

oder daß man eine sonst der Formel I entsprechende Verbindung, die jedoch an Stelle eines oder mehrerer

Wasserstoffatome eine oder mehrere reduzierbare Gruppe(n) und/oder eine oder mehrere zusätzliche C-C- und/oder C-N-Bindung(en) enthält, mit einem reduzierenden Mittel behandelt
oder daß man eine sonst der Formel I entsprechende Verbindung, die jedoch an Stelle eines oder mehrerer Wasserstoffatome eine oder mehrere solvolysierbare Gruppe(n) enthält, mit einem solvolysierenden Mittel behandelt
oder daß man zur Herstellung von Thioethern der Formel I, worin Q $-CH_2-S-CH_2CH_2-$ bedeutet, eine Verbindung der Formel IV
$Ind-CH_2N(R)_2$ IV
worin
R Alkyl mit 1- 4 C-Atomen, beide Reste R zusammen auch $-(CH_2)_p-$ oder $-CH_2CH_2OCH_2CH_2-$ und
p 4 oder 5
bedeuten und
Ind die angegebene Bedeutung hat
mit einem Thiol der Formel V

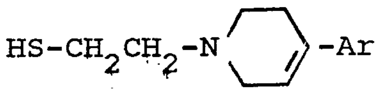

V

worin
Ar die angegebene Bedeutung hat
oder einem seiner reaktionsfähigen Derivate umsetzt
oder daß man eine Verbindung der Formel VI

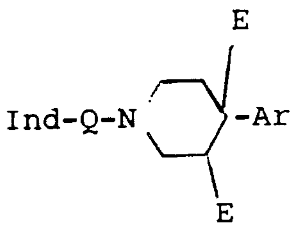

VI

worin
der eine Rest E X, CN oder $NH_2$, der andere Rest E H bedeutet und Ind, Q, Ar und X die angegebenen Bedeutungen haben,
mit einem HE-abspaltenden Mittel behandelt
oder daß man zur Herstellung einer Verbindung der Formel I, worin Ind einen durch $-O-CH_2-CO-R^1$ substituierten Indol-3-ylrest bedeutet, ein Hydroxyindol, das sonst der Formel I entspricht, aber an Stelle von Ind einen durch eine OH-Gruppe substituierten Indol-3-ylrest enthält, oder eines seiner reaktionsfähigen Derivate mit einer Verbindung der Formel $X-CH_2-CO-R^1$ (worin X und $R^1$ die angegebenen Bedeutungen haben) umsetzt
oder daß man zur Herstellung einer Verbindung der Formel I, worin Ind einen durch $-CO-NR^3R^4$ substituierten Indol-3-ylrest bedeutet, eine Indolcarbonsäure, die sonst der Formel I entspricht, aber an Stelle von Ind einen durch eine COOH-Gruppe substituierten Indol-3-ylrest enthält, oder eines ihrer reaktionsfähigen Derivate mit einer Verbindung der Formel $HNR^3R^4$ (worin $R^3$ und $R^4$ die angegebenen Bedeutungen haben) umsetzt
oder daß man zur Herstellung einer Verbindung der Formel I, worin Ind einen durch $-CS-NH_2$ substituierten Indol-3-ylrest bedeutet, ein Cyanindol, das sonst der Formel I entspricht, aber an Stelle von Ind einen durch eine CN-Gruppe substituierten Indol-3-ylrest enthält, mit $H_2S$ oder einem $H_2S$-abgebenden Mittel umsetzt
und/oder daß man gegebenenfalls in einer Verbindung der Formel I eine Thioethergruppe zu einer SO-Gruppe oder $SO_2$-Gruppe oder eine SO-Gruppe zu einer $SO_2$-Gruppe oxydiert und/oder eine OA-Gruppe unter Bildung einer OH-Gruppe spaltet und/oder eine Gruppe Ind in eine andere Gruppe Ind umwandelt und/oder daß man eine erhaltene Base der Formel I durch Behandeln mit einer Säure oder Base in eines ihrer Salze umwandelt.

Die Herstellung der Verbindungen der Formel I erfolgt im übrigen nach an sich bekannten Methoden, wie sie in der Literatur (z.B. in den Standardwerken wie Houben-Weyl, Methoden der Organischen Chemie,

Georg-Thieme-Verlag, Stuttgart; Organic Reactions, John Wiley & Sons, Inc., New York; DE-OS 33 42 632) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe für das beanspruchte Verfahren können gewünschtenfalls auch in situ gebildet werden, derart, daß man sie aus dem Reaktionsgemisch nicht isoliert, sondern sofort weiter zu den Verbindungen der Formel I umsetzt.

In den Indolderivaten der Formel II ist $X^1$ vorzugsweise X; dementsprechend sind in den Verbindungen der Formel III $X^2$ und $X^3$ vorzugsweise zusammen NH. Der Rest X ist vorzugsweise Cl oder Br; er kann jedoch auch J, OH oder eine reaktionsfähig funktionell abgewandelte OH-Gruppe bedeuten, insbesondere Alkylsulfonyloxy mit 1-6 (z.B. Methansulfonyloxy) oder Arylsulfonyloxy mit 6-10 C-Atomen (z.B. Benzolsulfonyloxy, p-Toluolsulfonyloxy, 1- oder 2-Naphthalin-sulfonyloxy).

Dementsprechend sind die Indolderivate der Formel I insbesondere durch Umsetzung von Verbindungen der Formel Ind-Q-Cl oder Ind-Q-Br mit Tetrahydropyridinderivaten der Formel III, worin $X^2$ und $X^3$ zusammen eine NH-Gruppe bedeuten (nachstehend als IIIa bezeichnet) erhältlich.

Die Verbindungen der Formeln II und insbesondere III sind zum Teil bekannt; die nicht bekannten Verbindungen der Formeln II und III können leicht analog zu den bekannten Verbindungen hergestellt werden. Verbindungen der Formel II (Q = $-CH_2-S-CH_2CH_2-$) können z.B. hergestellt werden aus Mannich-Basen der Formel IV und Thiolen der Formel $HS-CH_2CH_2-X^1$, z.B. $HS-CH_2CH_2OH$. Die Sulfoxide und Sulfone der Formel II (Q = $-CH_2-SO-CH_2CH_2-$ oder $-CH_2-SO_2-CH_2CH_2-$) sind durch Oxydation der Thioether (II, Q = $-CH_2-S-CH_2CH_2-$) zugänglich. Primäre Alkohole der Formel Ind-Q-OH sind z.B. durch Reduktion der entsprechenden Carbonsäuren oder ihrer Ester erhältlich. Behandeln mit Thionylchlorid, Bromwasserstoff, Phosphortribromid oder ähnlichen Halogenverbindungen liefert die entsprechenden Halogenide der Formel Ind-Q-Hal. Die entsprechenden Sulfonyloxyverbindungen sind erhältlich aus den Alkoholen Ind-Q-OH durch Umsetzung mit den entsprechenden Sulfonsäurechloriden.

Die Jodverbindungen der Formel Ind-Q-J sind z.B. durch Einwirkung von Kaliumjodid auf die zugehörigen p-Toluolsulfonsäureester erhältlich. Die Amine der Formel $Ind-Q-NH_2$ sind z.B. aus den Halogeniden mit Phthalimidkalium oder durch Reduktion der entsprechenden Nitrile herstellbar.

Die Piperidinderivate IIIa sind größtenteils bekannt (vgl. DE-OS 20 60 816) und z.B. erhältlich durch Umsetzung von 4-Piperidon mit metallorganischen Verbindungen der Formel M-Ar (worin M ein Li-Atom oder MgHal bedeutet), anschließende Hydrolyse zu den entsprechenden 4-Ar-4-hydroxy-piperidinen sowie gewünschtenfalls nachfolgende Dehydratisierung zu 4-Ar-3,4-dehydro-piperidinen. Verbindungen der Formel III ($X^2$ und $X^3$ = jeweils X) sind z.B. herstellbar durch Reduktion von Diestern der Formel $AlkylOOC-CH_2-CAr=CH-COOAlkyl$ zu Diolen der Formel $HO-CH_2CH_2-CAr=CH-CH_2OH$ (III, $X^2$ = $X^3$ = OH) und gegebenenfalls anschließende Umsetzung mit $SOCl_2$ bzw. $PBr_3$.

Die Umsetzung der Verbindungen II und III verläuft nach Methoden, wie sie für die Alkylierung von Aminen aus der Literatur bekannt sind. Man kann ohne Gegenwart eines Lösungsmittels die Komponenten miteinander verschmelzen, gegebenenfalls im geschlossenen Rohr oder im Autoklaven. Es ist aber auch möglich, die Verbindungen in Gegenwart eines indifferenten Lösungsmittels umzusetzen. Als Lösungsmittel eignen sich z.B. Kohlenwasserstoffe, wie Benzol, Toluol, Xylol; Ketone wie Aceton, Butanon; Alkohole wie Methanol, Ethanol, Isopropanol, n-Butanol; Ether wie Tetrahydrofuran (THF) oder Dioxan; Amide wie Dimethylformamid (DMF) oder N-Methyl-pyrrolidon; Nitrile wie Acetonitril, gegebenenfalls auch Gemische dieser Lösungsmittel untereinander oder Gemische mit Wasser. Der Zusatz eines säurebindenden Mittels, beispielsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triethylamin, Dimethylanilin, Pyridin oder Chinolin oder eines Überschusses der Aminkomponente $Ind-Q-NH_2$ bzw. des Piperidinderivates der Formel IIIa kann günstig sein. Die Reaktionszeit liegt je nach den angewendeten Bedingungen zwischen einigen Minuten und 14 Tagen, die Reaktionstemperatur zwischen etwa 0 und 150°, normalerweise zwischen 20 und 130°.

Es ist ferner möglich, eine Verbindung der Formel I zu erhalten, indem man ein Vorprodukt, das an Stelle von Wasserstoffatomen eine oder mehrere reduzierbare Gruppe(n) und/oder eine oder mehrere zusätzliche C-C-und/oder C-N-Bindung(en) enthält, mit einem reduzierenden Mittel behandelt, vorzugsweise bei Temperaturen zwischen -80 und +250° in Gegenwart mindestens eines inerten Lösungsmittels.

Reduzierbare (durch Wasserstoff ersetzbare) Gruppen sind insbesondere Sauerstoff in einer Carbonylgruppe, Hydroxyl, Arylsulfonyloxy (z.B. p-Toluolsulfonyloxy), N-Benzolsulfonyl, N-Benzyl oder O-Benzyl.

Es ist grundsätzlich möglich, Verbindungen, die nur eine, oder solche, die nebeneinander zwei oder mehr der oben angeführten Gruppen bzw. zusätzlichen Bindungen enthalten, reduktiv in eine Verbindung der Formel I überzuführen; dabei können gleichzeitig Substituenten in der Gruppe Ind, die in der

Ausgangsverbindung enthalten sind, reduziert werden. Vorzugsweise bedient man sich hierzu des nascierenden Wasserstoffs oder komplexer Metallhydride, ferner der Reduktion nach Wolff-Kishner.

Bevorzugte Ausgangsstoffe für die Reduktion entsprechen der Formel VII

Ind′-L-W VII

worin

Ind′ einen Rest Ind, der zusätzlich durch eine Arylsulfonylgruppe oder eine Benzylgruppe in 1-Stellung substituiert sein kann,

L Q oder eine dem Rest Q entsprechende Kette, worin jedoch eine oder mehrere $-CH_2$-Gruppe(n) durch -CO-und/oder ein oder mehrere Wasserstoffatome durch OH-Gruppen ersetzt sind,

W 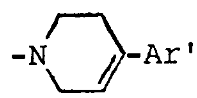 oder 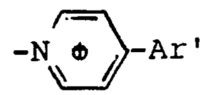 $An^{\ominus}$;

$An^{\ominus}$ ein Anion einer starken Säure und

Ar′ eine unsubstituierte oder ein- oder zweifach durch F, Cl, Br, OA, OH und/oder O-Benzyl oder durch eine Methylendioxygruppe substituierte Phenylgruppe bedeuten,

worin jedoch nicht gleichzeitig Ind′ = Ind, L = Q und

W = 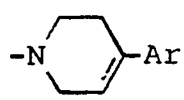

sein können.

In den Verbindungen der Formel VII ist L bevorzugt $-CO-(CH_2)_{n-2}-CO-$ [im einzelnen -COCO-, $-CO CH_2CO-$, $-CO-(CH_2)_2CO-$, $-CO-(CH_2)_3-CO-$], $-(CH_2)_{n-1}-CO-$[im einzelnen $-CH_2CO-$, $-CH_2CH_2-CO-$, $-(CH_2)_3-CO-$ oder $-(CH_2)_4-CO-$], $-CH_2-S-CH_2-CO-$, $-CH_2-SO-CH_2-CO-$ oder $-CH_2-SO_2-CH_2-CO-$, ferner z.B. $-CO-CH_2CH_2-$, $-CH_2-CO-CH_2-$, $-CO-(CH_2)_3-$, $-CH_2-CO-CH_2CH_2-$, $-CH_2 CH_2-CO-CH_2-$, $-CO-(CH_2)_4-$, $-CH_2-CO-(CH_2)_3-$, $-CH_2CH_2-CO-CH_2CH_2-$ oder $-(CH_2)_3-CO-CH_2-$.

Verbindungen der Formel VII sind z.B. herstellbar durch Umsetzung von 4-Ar′-1,2,3,6-tetrahydropyridin oder 4-Ar′-pyridin mit einer Verbindung der Formel VIII

Ind′-L-$X^1$ VIII

worin

Ar′, Ind′, L und $X^1$ die oben angegebenen Bedeutungen haben,

unter den Bedingungen, die oben für die Umsetzung von II mit III angegeben sind.

Wird als Reduktionsmittel nascierender Wasserstoff verwendet, so kann man diesen z.B. durch Behandlung von Metallen mit schwachen Säuren oder mit Basen erzeugen. So kann man z.B. ein Gemisch von Zink mit Alkalilauge oder von Eisen mit Essigsäure verwenden. Geeignet ist auch die Verwendung von Natrium oder einem anderen Alka limetall in einem Alkohol wie Ethanol, Isopropanol, Butanol, Amyl- oder Isoamylalkohol oder Phenol. Man kann ferner eine Aluminium-Nickel-Legierung in alkalisch-wässeriger Lösung, gegebenenfalls unter Zusatz von Ethanol, verwenden. Auch Natrium- oder Aluminiumamalgam in wässerig-alkoholischer oder wässeriger Lösung sind zur Erzeugung des nascierenden Wasserstoffs geeignet. Die Umsetzung kann auch in heterogener Phase durchgeführt werden, wobei man zweckmäßig eine wässerige und eine Benzol- oder Toluol-Phase verwendet.

Als Reduktionsmittel können ferner besonders vorteilhaft komplexe Metallhydride, wie $LiAlH_4$, $NaBH_4$ Diisobutylaluminiumhydrid oder $NaAl(OCH_2CH_2OCH_3)_2H_2$ sowie Diboran eingesetzt werden, falls erwünscht unter zusatz von Katalysatoren wie $BF_3$, $AlCl_3$ oder LiBr. Als Lösungsmittel eignen sich hierfür insbesondere Ether wie Diethylether, Di-n-butylether, THF, Dioxan, Diglyme oder 1,2-Dimethoxyethan sowie Kohlenwasserstoffe wie Benzol. Für eine Reduktion mit $NaBH_4$ sind in erster Linie Alkohole wie Methanol oder Ethanol, ferner Wasser sowie wässerige Alkohole als Lösungsmittel geeignet. Nach diesen Methoden reduziert man vorzugsweise bei Temperaturen zwischen -80 und +150°, insbesondere zwischen etwa 0 und etwa 100°.

Besonders vorteilhaft lassen sich -CO-Gruppen in Säureamiden (z.B. solchen der Formel VII, worin L eine $-(CH_2)_{n-1}-CO-$ oder $-CH_2-S-CH_2-CO-$Gruppe bedeutet) mit $LiAlH_4$ in THF bei Temperaturen zwischen etwa 0 und 66° zu $CH_2$-Gruppen reduzieren. Dabei können in 1-Stellung des Indolring befindliche Arylsulfonyl-Schutzgruppen gleichzeitig reduktiv abgespalten werden.

Eine Reduktion der Pyridiniumsalze der Formel VII

(worin W 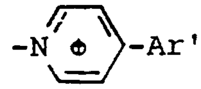 $An^{\ominus}$ und An

vorzugsweise Cl, Br oder $CH_3SO_3$ bedeutet) zu Verbindungen der Formel I gelingt z.B. mit $NaBH_4$ in Wasser, Methanol oder Ethanol oder in Gemischen dieser Lösungsmittel, falls erwünscht unter Zusatz einer Base wie NaOH, bei Temperaturen zwischen etwa 0 und 80°.

N-Benzylgruppen können reduktiv mit Natrium in flüssigem Ammoniak abgespalten werden.

Es ist ferner möglich, eine oder mehrere Carbonylgruppen nach der Methode von Wolff-Kishner zu $CH_2$-Gruppen zu reduzieren, z.B. durch Behandlung mit wasserfreiem Hydrazin in absolutem Ethanol unter Druck bei Temperaturen zwischen etwa 150 und 250°. Als Katalysator wird vorteilhaft Natriumalkoholat verwendet. Die Reduktion kann auch nach der Methode von Huang-Minlon variiert werden, indem man mit Hydrazinhydrat in einem hochsiedenden, mit Wasser mischbaren Lösungsmittel, wie Diethylenglykol oder Triethylenglykol, in Gegenwart von Alkali, wie Natriumhydroxid, umsetzt. Das Reaktionsgemisch wird in der Regel etwa 3-4 Stunden gekocht. Anschließend wird das Wasser abdestilliert und das gebildete Hydrazon bei Temperaturen bis zu etwa 200° zersetzt. Die Wolff-Kishner-Reduktion kann auch bei Raumtemperatur in Dimethylsulfoxid mit Hydrazin ausgeführt werden.

Verbindungen, die sonst der Formel I entsprechen, aber an Stelle eines oder mehrerer H-Atome eine oder mehrere solvolysierbare Gruppe(n) enthalten, können zu den Verbindungen der Formel I solvolysiert, insbesondere hydrolysiert werden.

Die Ausgangsstoffe für die Solvolyse sind beispielsweise erhältlich durch Reaktion von IIIa mit Verbindungen, die der Formel II ($X^1$ = X) entsprechen, aber an Stelle eines oder mehrerer H-Atome eine oder mehrere solvolysierbare Gruppe(n) enthalten. So können insbesondere 1-Acylindolderivate (entsprechend der Formel I, aber in 1-Stellung des Ind-Rests eine Acylgruppe enthaltend, vorzugsweise eine Alkanoyl-, Alkylsulfonyl-oder Arylsulfonylgruppe mit jeweils bis zu 10 C-Atomen, wie Methan-, Benzol- oder p-Toluolsulfonyl) zu den entsprechenden in der 1-Stellung des Indolringes unsubstituierten Indolderivaten hydrolysiert werden, z.B. in saurem, besser in neutralem oder alkalischem Medium bei Temperaturen zwischen 0 und 200°. Als Basen verwendet man zweckmäßig Natrium-, Kalium- oder Calciumhydroxid, Natrium- oder Kaliumcarbonat, oder Ammoniak. Als Lösungsmittel wählt man vorzugsweise Wasser; niedere Alkohole wie Methanol, Ethanol; Ether wie THF, Dioxan; Sulfone wie Tetramethylensulfon; oder deren Gemische, besonders die Wasser enthaltenden Gemische. Eine Hydrolyse kann auch bereits beim Behandeln mit Wasser allein erfolgen, insbesondere in der Siedehitze.

Indolderivate der Formel I (Q = $-CH_2-S-CH_2CH_2-$) sind ferner durch Umsetzung von Mannich-Basen der Formel IV mit Thiolen der Formel V oder ihren Salzen oder reaktionsfähigen Derivaten erhältlich.

Die Ausgangsstoffe der Formeln IV und V sind teilweise bekannt; die nicht bekannten unter diesen Ausgangsstoffen können leicht analog zu den bekannten Verbindungen hergestellt werden. So sind die Mannich-Basen der Formel IV z.B. aus Indolen der Formel Ind-H, Formaldehyd und Aminen der Formel HN-(R )$_2$ erhältlich, die Thiole der Formel V aus den Basen der Formel IIIa und Thiolderivaten der Formel HS-$CH_2CH_2$-$X^1$ (wobei die HS-Gruppe auch intermediär geschützt werden kann).

Im einzelnen erfolgt die Umsetzung IV mit V in Gegenwart oder Abwesenheit eines inerten Lösungsmittels bei Temperaturen zwischen etwa -20 und 250°, vorzugsweise zwischen 60 und 150°. Als Lösungsmittel eignen sich beispielsweise Kohlenwasserstoffe wie Benzol, Toluol, Xylole oder Mesitylen; tertiäre Basen wie Triethylamin; Pyridin oder Picoline; Alkohole wie Methanol, Ethanol oder Butanol; Glykole und Glykolether wie Ethylenglykol, Diethylenglykol, 2-Methoxy-ethanol; Ketone wie Aceton; Ether wie THF oder Dioxan; Amide wie DMF; Sulfoxide wie Dimethylsulfoxid. Auch Gemische dieser Lösungsmittel sind geeignet. Die Thiole der Formel V werden zweckmäßig zunächst in die entsprechenden Mercaptide umgewandelt, vorzugsweise durch Reaktion mit Natrium- oder Kaliumhydroxid, Natrium- oder Kaliumethylat in die entsprechenden Natrium- oder Kaliummercaptide. Als reaktionsfähige Derivate der Thiole der Formel V lassen sich vorzugsweise die entsprechenden Isothioharnstoffe (entsprechend V, aber $H_2N(=NH)$-S- an Stelle von HS-) bzw. deren Salze (z.B. die Isothiuroniumchloride) verwenden; diese sind stabiler und leichter zu handhaben. Man arbeitet bei ihrer Umsetzung mit den Basen IV zweckmäßig in basischem Medium, z.B. in wässeriger Natronlauge, bei Temperaturen zwischen 0 und 100°.

Man gelangt ferner zu Verbindungen der Formel I, indem man aus Verbindungen der Formel VI unter Ausbildung einer Doppelbindung HE abspaltet. Entsprechend der Definition von E kann es sich z.B. handeln um eine Abspaltung von Halogenwasserstoff, Wasser (Dehydratisierung), einer Carbonsäure oder einer anderen Säure, von Ammoniak oder von HCN. Die Ausgangsstoffe der Formel VI sind z.B. erhältlich durch Umsetzung von II ($X^1$ = X) mit einer Verbindung der Formel IX

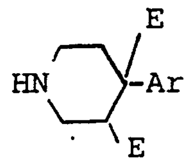

IX

worin E und Ar die angegebenen Bedeutungen haben.

Falls einer der Reste E = Hal ist, kann dieser Substituent unter basischen Reaktionsbedingungen leicht eliminiert werden. Als Basen können verwendet werden: Alkalimetallhydroxide, Alkalimetallcarbonate, Alkoholate, wie z.B. Kalium-tert.-butylat, Amine, wie z.B. Dimethylanilin, Pyridin, Collidin oder Chinolin; als Lösungsmittel benutzt man z.B. Benzol, Toluol, Cyclohexan, THF oder tert.-Butanol. Die als Basen verwendeten Amine können auch im Überschuß als Lösungsmittel eingesetzt werden. Bedeutet der eine der Reste E eine OH-Gruppe, so benutzt man als wasserabspaltende Mittel vorzugsweise Säuren wie Essigsäure, Salzsäure oder Gemische beider. Der Zusatz eines Lösungsmittels (z.B. Wasser oder Ethanol) kann von Vorteil sein. Die Eliminierung von Acyl-, Alkylsulfonyl-sowie Alkoxysulfonyl-oxy- oder Amino-Resten kann unter ähnlichen Bedingungen durchgeführt werden. Eine Eliminierung von Sulfonsäure-Resten, z.B. die der Mesylate oder Tosylate, erfolgt schonend durch Kochen in DMF oder Dimethylsulfoxid mit Alkalimetallcarbonaten, z.B. $Li_2CO_3$, oder mit Kaliumacetat. Ammoniak kann bereits durch Erhitzen der Salze der entsprechenden Aminoverbindungen (insbesondere der 4-Aminoderivate) abgespalten werden. In ähnlicher Weise kann HCN aus Verbindungen der Formel VI (eine Gruppe E = CN) durch Erhitzen abgespalten werden. Die Eliminierung von HE aus VI erfolgt allgemein bei Temperaturen zwischen 0 und etwa 250°, vorzugsweise zwischen 50 und 200°.

Verbindungen der Formel I, worin Ind einen durch $-O-CH_2-CO-R^1$ substituierten Indol-3-yl-rest bedeutet, können durch Veretherung entsprechender Hydroxyindol-3-yl-Verbindungen (vgl. EP-7399) oder ihrer reaktionsfähigen Derivate, insbesondere ihrer Salze, z.B. ihrer Alkalimetallsalze wie ihrer Na- oder K-Salze, mit Verbindungen der Formel $X-CH_2-CO-R^1$ (z.B. Chlor- oder Bromessigsäure, Chlor- oder Bromessigsäuremethyl- oder ethylester, Chlor-oder Bromacetamid, Chlor- oder Brom-N-methyl-acetamid, Chlor- oder Brom-N,N-dimethyl-acetamid) erhalten werden. Man arbeitet dabei zweckmäßig in einem der angegebenen Lösungsmittel (z.B. DMF), wobei man zunächst die Hydroxyverbindung mit Hilfe einer Base, z.B. eines Alkalimetallhydrids wie NaH oder KH oder eines Alkalimetallalkoholats wie Natrium- oder Kaliummethylat oder -ethylat, in eines ihrer Salze überführt, dann die Verbindung der Formel $X-CH_2-CO-R^1$ hinzufügt und anschließend einige Stunden bei Temperaturen zwischen 0 und 150°, vorzugsweise zwischen 20 und 80°, rührt.

Verbindungen der Formel I, worin Ind einen durch $-CO-NR^3R^4$ substituierten Indol-3-yl-rest bedeutet, können durch Amidierung entsprechender Carboxy-indol-3-yl-Verbindungen (vgl. DE-OS 33 42 632) oder ihrer reaktionsfähigen Derivate, z.B. ihrer Säurehalogenide, Ester oder Anhydride, mit Aminen der Formel $HNR^3R^4$ erhalten werden. Bevorzugt ist die Umsetzung der freien Carbonsäure mit dem Amin unter den Bedingungen einer Peptidsynthese. Diese Reaktion gelingt vorzugsweise in Gegenwart eines Dehydratisierungsmittels, z.B. eines Carbodiimids wie Dicyclohexylcarbodiimid oder N-(3-Dimethylaminopropyl)-N-ethylcarbodiimid, ferner Propanphosphonsäureanhydrid (vgl. Angew. Chem. 92, 129 (1980)), Diphenylphosphorylazid oder 2- Ethoxy-N-ethoxycarbonyl-1,2-dihydrochinolin, in einem inerten Lösungsmittel, z.B. einem halogenierten Kohlenwasserstoff wie Dichlormethan, einem Ether wie THF oder Dioxan, einem Amid wie DMF oder Dimethylacetamid, einem Nitril wie Acetonitril, bei Temperaturen zwischen etwa -10 und 40, vorzugsweise zwischen 0 und 30°. An Stelle der Säure bzw. des Amids können auch reaktionsfähige Derivate dieser Stoffe in die Reaktion eingesetzt werden, z.B. solche, in denen reaktive Gruppen intermediär durch Schutzgruppen blockiert sind. Die Säuren können auch in Form ihrer aktivierten Ester verwendet werden, die zweckmäßig in situ gebildet werden, z.B. durch Zusatz von 1-Hydroxybenztriazol oder N-Hydroxysuccinimid.

Verbindungen der Formel I, worin Ind einen durch $-CS-NH_2$ substituierten Indol-3-yl-rest bedeutet, können durch Anlagerung von $H_2S$ an entsprechende Cyan-indol-3-yl-Verbindungen erhalten werden. An Stelle von $H_2S$ können auch $H_2S$-abgebende Mittel verwendet werden, z.B. Thioacetamid. Man arbeitet zweckmäßig in einem der angegebenen Lösungsmittel, z.B. DMF, in Gegenwart einer Säure, z.B. HCl, bei Temperaturen zwischen 0 und 200, vorzugsweise zwischen 20 und 160°.

Weiterhin kann man eine Verbindung der Formel I nach an sich bekannten Methoden in eine andere Verbindung der Formel I umwandeln.

So kann man in einem Thioether der Formel I ($Q = -CH_2-S-CH_2CH_2-$) die Thioethergruppe zu einer SO-Gruppe oder zu einer $SO_2$-Gruppe oder in einem Sulfoxid der Formel I ($Q = -CH_2-SO-CH_2CH_2-$) die

SO-Gruppe zu einer $SO_2$-Gruppe oxydieren. Will man die Sulfoxide erhalten, so oxydiert man beispielsweise mit Wasserstoffperoxid, Persäuren wie m-Chlorperbenzoesäure, Cr(VI)-Verbindungen wie Chromsäure, $KMnO_4$, 1-Chlorbenztriazol, Ce(IV)-Verbindungen wie $(NH_4)_2Ce(NO_3)_6$, negativ substituierten aromatischen Diazoniumsalzen wie o- oder p-Nitrophenyldiazoniumchlorid oder elektrolytisch unter verhältnismäßig milden Bedingungen und bei relativ niedrigen Temperaturen (etwa -80 bis +100°). Will man dagegen die Sulfone (aus den Thioethern oder den Sulfoxiden) erhalten, so verwendet man die gleichen Oxydationsmittel unter kräftigeren Bedingungen und/oder im Überschuß sowie in der Regel bei höheren Temperaturen. Bei diesen Umsetzungen können die üblichen inerten Lösungsmittel zugegen oder abwesend sein. Als inerte Lösungsmittel eignen sich beispielsweise Wasser, wässerige Mineralsäuren, wässerige Alkalilaugen, niedere Alkohole wie Methanol oder Ethanol, Ester wie Ethylacetat, Ketone wie Aceton, niedere Carbonsäuren wie Essigsäure, Nitrile wie Acetonitril, Kohlenwasserstoffe wie Benzol, chlorierte Kohlenwasserstoffe wie Chloroform oder $CCl_4$. Ein bevorzugtes Oxydationsmittel ist 30 %iges wässeriges Wasserstoffperoxid. Dieses führt bei Anwendung der berechneten Menge in Lösungsmitteln wie Essigsäure, Aceton, Methanol, Ethanol oder wässeriger Natronlauge bei Temperaturen zwischen -20 und 100° zu den Sulfoxiden, im Überschuß bei höheren Temperaturen, vorzugsweise in Essigsäure oder in einem Gemisch aus Essigsäure und Acetanhydrid, zu den Sulfonen.

Ether der Formel I, in denen der Rest Ar ein- oder zweifach durch O-Alkyl substituiert ist, können gespalten werden, wobei die entsprechenden Hydroxyderivate entstehen. Z.B. kann man die Ether spalten durch Behandeln mit Dimethylsulfid-Bortribromid-Komplex, z.B. in Toluol, Ethern wie THF oder Dimethylsulfoxid, oder durch Verschmelzen mit Pyridin- oder Anilin-hydrohalogeniden, vorzugsweise Pyridinhydrochlorid, bei etwa 150-250°.

Weiterhin kann man nach an sich bekannten Methoden Ind-Gruppen in andere Ind-Gruppen umwandeln. Carboxylgruppen können verestert werden, z.B. durch Behandeln mit Alkoholen in Gegenwart von sauren Katalysatoren oder durch Reaktion mit Diazoalkanen. Umwandlung der Carbonsäuren in ihre Chloride, z.B. mit $SOCl_2$, und nachfolgende Reaktion mit $NH_3$ oder Aminen führt zu den entsprechenden Carboxamiden, die auch durch Behandeln der Carbonsäureester mit Ammoniak oder Aminen erhältlich sind. Eine Solvolyse, vorzugsweise eine Hydrolyse unter den oben angegebenen Bedingungen, führt von den Estern oder Amiden zu den Carbonsäuren; insbesondere sind Carbonsäuren aus den Estern erhältlich, in dem man diese mit NaOH oder KOH in wässerigen Alkoholen behandelt, zweckmäßig bei Temperaturen zwischen etwa 50 und etwa 200°. Eine Reduktion von Nitrogruppen zu Aminogruppen gelingt z.B. mit Metallen wie Fe, Sn oder Zn oder mit $SnCl_2$, zweckmäßig in wässerigalkoholischen Säuren, z.B. in wässerig-ethanolischer Salzsäure bei Temperaturen zwischen 20 und 100°. Hydroxygruppen oder Aminogruppen können acyliert werden, z.B. mit Säurechloriden wie Acetylchlorid, Benzoylchlorid oder Methansulfonylchlorid in Gegenwart einer Base wie Triethylamin oder Pyridin in Anwesenheit oder Abwesenheit eines der angegebenen Lösungmittel. Reaktionen von Hydroxyverbindungen mit Alkylisocyanaten, z.B. in Gegenwart einer Base wie Pyridin, führen zu den entsprechenden Urethanen.

Die Verbindungen der Formel I können ein oder mehrere Asymmetriezentren besitzen. Sie können daher bei ihrer Herstellung als Racemate oder, falls optisch aktive Ausgangsstoffe verwendet werden, auch in optisch aktiver Form erhalten werden. Weisen die Verbindungen zwei oder mehr Asymmetriezentren auf, dann fallen sie bei der Syn these im allgemeinen als Gemische von Racematen an, aus denen man die einzelnen Racemate, beispielsweise durch Umkristallisieren aus inerten Lösungsmitteln, in reiner Form isolieren kann. Erhaltene Racemate können, falls erwünscht, nach an sich bekannten Methoden mechanisch oder chemisch in ihre optischen Antipoden getrennt werden. Vorzugsweise werden aus dem Racemat durch Umsetzung mit einem optisch aktiven Trennmittel Diastereomere gebildet. Als Trennmittel eignen sich z.B. optisch aktive Säuren, wie die D- und L-Formen von Weinsäure, Dibenzoylweinsäure, Diacetylweinsäure, Camphersulfonsäuren, Mandelsäure, Äpfelsäure oder Milchsäure. Die verschiedenen Formen der Diastereomeren können in an sich bekannter Weise, z.B. durch fraktionierte Kristallisation, getrennt, und die optisch aktiven Verbindungen der Formel I können in an sich bekannter Weise aus den Diastereomeren in Freiheit gesetzt werden.

Eine erhaltene Base der Formel I kann mit einer Säure in das zugehörige Säureadditionssalz übergeführt werden. Für diese Umsetzung eignen sich Säuren, die physiologisch unbedenkliche Salze liefern. So können anorganische Säuren verwendet werden, z.B. Schwefelsäure, Halogenwasserstoffsäuren wie Chlorwasserstoffsäure oder Bromwasserstoffsäure, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-Phenylpropion säure, Citronensäure, Gluconsäure, Ascorbinsäure, Nicotinsäure, Isonicotinsäure, Methan- oder

Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden.

Gegenstand der Erfindung ist ferner die Verwendung der Verbindungen der Formel I und ihrer physiologisch unbedenklichen Salze zur Herstellung pharmazeutischer Zübereitungen, insbesondere auf nicht-chemischem Wege. Hierbei können sie zusammen mit mindestens einem Träger-oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zübereitungen, enthaltend mindestens eine Verbindung der Formel I und/oder eines ihrer physiologisch unbedenklichen Salze. Diese Zübereitungen können als Arzneimittel in der Human- oder Veterinärmedizin eingesetzt werden. Als Trägersubstanzen kommen organische oder anorganische Stoffe in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyethylenglykole, Gelatine, Kohlehydrate wie Lactose oder Stärke, Magnesiumstearat, Talk, Vaseline. Zur enteralen Applikation dienen insbesondere Tabletten, Dragees, Kapseln, Sirupe, Säfte, Tropfen oder Suppositorien, zur parenteralen Applikation Lösungen, vorzugsweise ölige oder wässerige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden.

Die angegebenen Zübereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Die Verbindungen der Formel I und ihre physiologisch unbedenklichen Salze können bei der therapeutischen Behandlung des menschlichen oder tierischen Körpers und bei der Bekämpfung von Krankheiten verwendet werden, insbesondere von Parkinsonismus, von extrapyramidalen Störungen bei der Neuroleptikatherapie, von Depressionen und/oder Psychosen und von Nebenwirkungen bei der Behandlung der Hypertonie (z.B. mit $\alpha$-Methyldopa). Ferner können die Verbindungen in der Endokrinologie und Gynäkologie Verwendung finden, z.B. zur Therapie von Akromegalie, Hypogonadismus, sekundärer Amenorrhoe, prämenstruellem Syndrom, unerwünschter puerperaler Laktation und generell als Prolaktin-Hemmer, weiterhin zur Therapie cerebraler Störungen (z.B. Migräne), insbesondere in der Geriatrie ähnlich wie gewisse Ergot-Alkaloide.

Dabei werden die erfindungsgemäßen Substanzen in der Regel in Analogie zu bekannten, im Handel befindlichen Präparaten (z.B. Bromocriptin, Dihydroergocornin) verabreicht, vorzugsweise in Dosierungen zwischen etwa 0,2 und 500 mg, insbesondere zwischen 0,2 und 50 mg pro Dosierungseinheit. Die tägliche Dosierung liegt vorzugsweise zwischen etwa 0,001 und 10 mg/kg Körpergewicht. Die niedrigen Dosierungen (etwa 0,2 bis 1 mg pro Dosierungseinheit; etwa 0,001 bis 0,005 mg/kg Körpergewicht) kommen dabei insbesondere für die Verwendung als Migränemittel in Betracht; für die übrigen Indikationen werden Dosierungen zwischen 10 und 50 mg pro Dosierungseinheit bevorzugt. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

In den nachstehenden Beispielen bedeutet "übliche Aufarbeitung": Man gibt, falls erforderlich, Wasser hinzu, extrahiert mit Dichlormethan, trennt ab, trocknet die organische Phase über Natriumsulfat, filtriert, dampft ein und reinigt durch Chromatographie an Kieselgel und/oder durch Kristallisation. Temperaturen sind in °C angegeben. IR = Hauptbanden des IR-Spektrums (KBr). MS = Peaks des Massenspektrums. Rf-Werte wurden dünnschichtchromatographisch an Kieselgel erhalten.

Beispiel 1

Man rührt eine Lösung von 26,6 g 3-(4-Chlorbutyl)-5-indolyl-harnstoff [erhältlich durch Umsetzung von 5-Nitroindol mit 4-Chlorbutyrylchlorid zu 3-(4-Chlorbutyryl)-5-nitro indol, Reduktion mit Diboran zu 3-(4-Chlorbutyl)-5-nitroindol, Hydrierung zu 3-(4-Chlorbutyl)-5-amino-indol und Umsetzung mit KCNO] und 16 g 4-Phenyl-1,2,3,6-tetrahydropyridin in 200 ml Acetonitril 12 Std. bei 20°, arbeitet wie üblich auf und erhält 3-[4-(4-Phenyl-1,2,3,6-tetrahydro-1-pyridyl)-butyl]-5-indolyl-harnstoff, Dihydrat, F. 158° (Zers.).

Analog erhält man aus den entsprechenden Ausgangsstoffen der Formeln II und III:

3-[4-(4-Phenyl-1,2,3,6-tetrahydro-1-pyridyl)-butyl]-5-nitro-indol, F. 148-150°

3-[4-(4-p-Methoxyphenyl-1,2,3,6-tetrahydro-1-pyridyl)butyl]-5-indolylharnstoff

3-[4-(4-(3,4-Dimethoxyphenyl)-1,2,3,6-tetrahydro-1-pyridyl)-butyl]-5-indolylharnstoff

3-[4-(4-(3,4-Methylendioxyphenyl)-1,2,3,6-tetrahydro-1-pyridyl)-butyl]-5-indolylharnstoff

3-[4-(4-(2-Thienyl)-1,2,3,6-tetrahydro-1-pyridyl)-butyl]-5-indolylharnstoff

3-[4-(4-(3-Thienyl)-1,2,3,6-tetrahydro-1-pyridyl)-butyl]-5-indolylharnstoff

3-[2-(4-Phenyl-1,2,3,6-tetrahydro-1-pyridyl)-ethyl]-5-indolyl-harnstoff

3-[3-(4-Phenyl-1,2,3,6-tetrahydro-1-pyridyl)-propyl]-5-indolyl-harnstoff

3-[5-(Phenyl-1,2,3,6-tetrahydro-1-pyridyl)-pentyl]-5-indolyl-harnstoff.

Beispiel 2

Ein Gemisch von 2,46 g 3-(4-Aminobutyl)-5-indolyl-harnstoff [erhältlich aus 5-Indolyl-harnstoff über 3-(4-Chlorbutyryl)-5-indolyl-harnstoff, 3-(4-Chlorbutyl)-5- indolyl-harnstoff und 3-(4-Phthalimidobutyl)-5-indolyl-harnstoff] und 2,15 g 1,5-Dichlor-3-phenyl-2-penten in 40 ml Aceton und 40 ml Wasser wird 24 Std. gekocht und wie üblich aufgearbeitet. Man erhält 3-[4-(4-Phenyl-1,2, 3,6-tetrahydro-1-pyridyl)-butyl]-5-indolyl-harnstoff, Dihydrat, F. 158° (Zers.).

Beispiel 3

Zu einer Suspension von 11,7 g $LiAlH_4$ in 1000 ml absolutem THF tropft man unter Rühren eine Suspension von 40,7 g 3-[4-(4-Phenyl-1,2,3,6-tetrahydro-1-pyridyl)-4-oxo-2-thia-butyl]-5-nitroindol [erhältlich aus 4-(5-Nitro-3-indolyl)-3-thiabuttersäure und 4-Phenyl-1,2,3,6-tetrahydropyridin] in 3 l absolutem THF, zersetzt mit Wasser und Natronlauge und arbeitet wie üblich auf. Man erhält 3-[4-(4-Phenyl-1,2,3,6-tetrahydro-1-pyridyl)-2-t hiabutyl]-5-nitro-indol, Hydrochlorid, F. 157-158°. (Die Nitrogruppe wird durch das Reduktionsmittel nicht angegriffen!).

Beispiel 4

Zu einer Lösung von 4,65 g 1-[4-(5-Ureido-3-indolyl)-butyl]-4-phenylpyridiniumbromid [erhältlich aus 3-(4-Brombutyl)-5-indolyl-harnstoff und 4-Phenylpyridin] in 50 ml 1 n NaOH gibt man unter Rühren 1 g $NaBH_4$ in 20 ml Wasser und rührt danach noch 3 Std. bei 60°. Nach üblicher Aufarbeitung erhält man 3-[4-(4-Phenyl-1,2,3,6-tetrahydro-1-pyridyl)-butyl]-5-indolyl-harnstoff, Dihydrat, F. 158° (Zers.).

Beispiel 5

Man kocht 4,68 g 1-Benzolsulfonyl-3-[4-(4-phenyl-1,2,3,6-tetrahydro-1-pyridyl)-butyl]-5-indolyl-harnstoff [erhältlich aus 1-Benzolsulfonyl-3-(4-chlorbutyl)-5-indolyl-harnstoff und 4-Phenyl-1,2,3,6-tetrahydropyridin] mit 1 g KOH in 7 ml Wasser und 14 ml Ethanol 16 Stunden, arbeitet wie üblich auf und erhält 3-[4-(4-Phenyl-1,2, 3,6-tetrahydro-1-pyridyl)-butyl]-5-indolyl-harnstoff, Dihydrat, F. 158° (Zers.).

Beispiel 6

Man löst 2,76 g Na in 180 ml Ethanol, gibt 21,9 g 1-(2-Mercaptoethyl)-4-phenyl-1,2,3,6-tetrahydropyridin und 23,2 g 5-Ureido-gramin hinzu, kocht 16 Std., dampft ein, arbeitet wie üblich auf und erhält 3-[4-(4-Phenyl-1,2, 3,6-tetrahydro-1-pyridyl)-2-thiabutyl]-5-indolyl-harnstoff, F. 158° (Zers.).

Beispiel 7

Ein Gemisch von 26,9 g 5-Ureido-gramin-hydrochlorid, 29,8 g S-[2-(4-Phenyl-1,2,3,6-tetrahydro-1-pyridyl)-ethyl]-isothiuroniumchlorid und 200 ml 1,5 n wässeriger Natronlauge wird 4 Std. bei 40° gerührt. Nach üblicher Aufarbeitung erhält man 3-[4-(4-Phenyl-1,2,3,6-tetrahydro-1-pyridyl)-2-thiabutyl]-5-indolyl-harnstoff, F. 158° (Zers.).

Analog erhält man mit 5-Amino-gramin-dihydrochlorid das 3-[4-(4-Phenyl-1,2,3,6-tetrahydro-1-pyridyl)-2-thiabutyl]-5-amino-indol, Rf 0,18 in Toluol/Methanol/Triethylamin (8 : 2 : 1).

Beispiel 8

Man erhitzt 4,07 g 3-[4-(4-Hydroxy-4-phenyl-1-piperidyl)-butyl]-5-nitro-indol [erhältlich durch Reaktion von 3-(4-Brombutyl)-5-nitro-indol mit 4-Piperidon, anschließßende Umsetzung mit $C_6H_5Li$ und Hydrolyse] mit 40 ml 2n Salzsäure 2 Std. auf 50°, arbeitet wie üblich auf und erhält 3-[4-(4-Phenyl-1,2,3,6-tetrahydro-1-pyridyl)-butyl]-5-nitro-indol, F. 148-150°.

Beispiel 9

Eine Lösung von 3,46 g 3-[4-(4-Phenyl-1,2,3,6-tetrahydro-1-pyridyl)-butyl]-5-hydroxy-indol in 35 ml DMF wird mit 0,24 g NaH (8o%ige Suspension in Mineralöl) versetzt und 1 Std. bei 20° gerührt. Anschließend gibt man eine Lösung von 0,94 g Chloracetamid in 10 ml DMF hinzu, erwärmt unter Rühren 2 Std. auf 50°, dampft ein, arbeitet wie üblich auf und erhält 3-[4-(4-Phenyl-1,2,3,6-tetrahydro-1-pyridyl)-butyl]-5-carbamoylmethoxy-indol, F. 94-96°. Methansulfonat, F. 193-195°.

Analog erhält man

3-[4-(4-Phenyl-1,2,3,6-tetrahydro-1-pyridyl)-butyl]-5-methylcarbamoylmethoxy-indol

3-[4-(4-Phenyl-1,2,3,6-tetrahydro-1-pyridyl)-butyl-5-dimethylcarbamoylmethoxy-indol, Hydrochlorid, F. 211° (Zers.)

3-[4-(4-Phenyl-1,2,3,6-tetrahydro-1-pyridyl)-butyl]-5-ethoxycarbonylmethoxy-indol, Rf 0,56 ($CH_2Cl_2/CH_3OH$, 9 : 1).

Beispiel 10

Eine Lösung von 3,74 g 3-[4-(4-Phenyl-1,2,3,6-tetrahydro-1-pyridyl)-butyl]-indol-5-carbonsäure in 50 ml DMF wird mit 1,01 g N-Methylmorpholin versetzt. Unter Rühren gibt man eine Lösung von 0,61 g 2-Aminoethanol in 5 ml DMF, 1,35 g 1-Hydroxybenztriazol sowie eine Lösung von 1,92 g N-(3-Dimethylaminopropyl)-N′-ethyl-carbodiimid-hydrochlorid in 20 ml DMF hinzu. Man rührt 16 Std. bei 20° und dampft das Filtrat ein. Nach üblicher Aufarbeitung erhält man 3-[4-(4-Phenyl-1,2,3,6-tetrahydro-1-pyridyl)-butyl]-indol-5-carbonsäure-N-(2-hydroxyethyl)-amid, F. 168-170°.

Analog erhält man mit den entsprechenden Aminen die folgenden Derivate der 3-[4-(4-Phenyl-1,2,3,6-tetrahydro-1-pyridyl)-butyl]-indol-5-carbonsäure:

-N-(3-hydroxypropyl)-amid, F. 154-158°

-N-(4-hydroxybutyl)-amid

-N-(1,1-dimethyl-2-hydroxyethyl)-amid, IR: 3419, 3265, 2927, 1638, 1620, 1579, 1522, 1471, 1448, 1368, 1308, 1129, 1061, 995, 963, 907, 857, 747, 693

-N-(2-hydroxyethyl)-N-methyl-amid, F. 142-145°

-N,N-bis-(2-hydroxyethyl)-amid, MS: 462, 444, 399, 375, 357, 327, 285, 257, 227, 198, 164, 158, 129, 105, 88, 74 -N-(2-methoxyethyl)-amid, F. 143-144°

-N-(2-acetoxyethyl)-amid

-N-(2-benzoyloxyethyl)-amid, MS: 399, 341, 241, 213, 173, 158, 122, 105, 91, 77

-N-(methoxycarbonylmethyl)-amid, F. 145-147°

-N-(ethoxycarbonylmethyl)-amid, F. 169°

-N-(carbamoylmethyl)-amid, Hydrat, F. 183-185°

-N-(2-sulfoethyl)-amid, F. 159-160°

-N-(2-dimethylaminoethyl)-amid, IR: 3417, 3240, 1632, 1578, 1535, 1495, 1466, 1445, 1375, 1304, 1244, 1185, 1128, 1097, 1034, 961, 908, 817, 747, 693

-anilid

-p-fluoranilid, F. 167-168°

-N-benzyl-amid, F. 155-156°

-N-benzyl-N-methyl-amid, IR: 3420, 3238, 3028, 2930, 2713, 1620, 1608, 1579, 1495, 1446, 1401, 1358, 1250, 1193, 1094, 1076, 1027, 1001, 817, 747, 697
-N-(2-morpholinoethyl)-amid, F. 153-154°
-N-[2-(2-pyridyl)-ethyl]-amid, F. 148-149°
-pyrrolidid, Dihydrochlorid, F: 188-190° (Zers.)
-piperidid, F. 94-95°
-morpholid, Dihydrochlorid, IR: 3415, 3227, 2925, 2858, 2710, 2588, 1621, 1495, 1440, 1386, 1362, 1329, 1273, 1248, 1192, 1156, 1113, 1068, 1023, 942, 920, 840, 819, 749, 606
-4-methyl-piperazid, IR: 3419, 3226, 3031, 2933, 2856, 2792, 1625, 1604, 1494, 1451, 1440, 1366, 1294, 1255, 1234, 1172, 1139, 1071, 1050, 1023, 964, 925, 892, 747, 693
-4-phenyl-piperazid, IR: 3412, 3231, 3038, 3002, 2919, 2854, 2765, 1626, 1593, 1565, 1479, 1457, 1434, 1377, 1311, 1281, 1238, 1160, 1132, 1098, 1052, 1014, 980, 945, 864, 813, 772, 747, 694
-4-(2-pyrimidinyl)-piperazid, Dihydrochlorid, F. 235-236° (Zers.)
-4-(5-methyl-2-thiazolyl)-piperazid, Dihydrochlorid, F. 211-213° (Zers.)
-4-p-fluorbenzoyl-piperazid, F. 143-145°
-4-ethoxycarbonyl-piperazid, F. 115-119° (Zers.)
-4-(carbamoylmethyl)-piperazid
-4-(N-isopropyl-carbamoylmethyl)-piperazid, Dihydrochlorid, F. 156° (Zers.)
-4-(N,N-dimethyl-carbamoylmethyl)-piperazid
-4-pyrrolidinocarbonylmethyl-piperazid, MS: 553, 457, 441, 394, 357, 327, 296, 227, 198, 155, 151, 129, 115, 91, 70, 56, 42
-4-dimethylamino-piperidid, Dihydrochlorid-Dihydrat, Zers. ab 105°
-4-p-fluorbenzamido-piperidid, F. 110° (Zers.)
-4-ethoxycarbonyl-piperidid, F. 117-119°
-4-carbamoyl-piperidid, F. 93-98°
-4-p-chlorphenyl-piperidid, IR: 3422, 3236, 3020, 2928, 2854, 1643, 1602, 1493, 1445, 1369, 1320, 1297, 1276, 1231, 1130, 1090, 1033, 1014, 826, 747, 694
-4-piperidino-piperidid, Dihydrochlorid, Rf 0,15 (Dichlormethan/Methanol/Ethylacetat 7:2:1)
-4-morpholino-piperidid, Dihydrochlorid-hydrat, F. 90° (Zers.)
-4-phenyl-1,2,3,6-tetrahydro-pyridid, Hydrochlorid, F. 88-89°.

Analog erhält man die folgenden Derivate der 3-[4-(4-Phenyl-1,2,3,6-tetrahydro-1-pyridyl)-butyl]-indol-5-oxyessigsäure:
-N-(ethoxycarbonyl)-amid, Hydrochlorid, F. 191-193°
-N-(1-ethoxycarbonyl-2-hydroxy-ethyl)-amid, Hydrochlorid, F. 118-120°.

Beispiel 11

In eine Lösung von 3,55 g 3-[4-(4-Phenyl-1,2,3,6-tetrahydro-1-pyridyl)-butyl]-5-cyan-indol und 1,5 g Thioacetamid in 40 ml DMF leitet man bis zur Sättigung HCl ein und kocht 30 Minuten. Man dampft ein, arbeitet wie üblich auf und erhält 3-[4-(4-Phenyl-1,2,3,6-tetrahydro-1-pyridyl)-butyl]-indol-5-thiocarboxamid, Zers. bei 118°.

Beispiel 12

Zu einer siedenden Lösung von 3,93 g 3-[4-(4-Phenyl-1,2,3,6-tetrahydro-1-pyridyl)-2-thiabutyl]-5-nitro-indol in 50 ml Ethanol gibt man 6 ml 30%iges $H_2O_2$ und kocht anschließend 3 Stunden. Nach Zugabe von weiteren 4 ml 30%igem $H_2O_2$ kocht man noch 9 Std., kühlt ab, arbeitet wie üblich auf und erhält 3-[4-(4-Phenyl-1,2,3,6-tetrahydro-1-pyridyl)-2-thiabutyl]-5-nitro-indol-S-oxid.

Beispiel 13

Zu einer Lösung von 3,93 g 3-[4-(4-Phenyl-1,2,3,6-tetrahydro-1-pyridyl)-2-thiabutyl]-5-nitro-indol in 20 ml Essigsäure gibt man 9 ml 30%iges $H_2O_2$ und kocht 90 Minuten. Nach üblicher Aufarbeitung erhält man 3-[4-(4-Phenyl-1,2,3,6-tetrahydro-1-pyridyl)-2-thiabutyl]-5-nitro-indol-S,S-dioxid, Rf 0,7 (Aceton).

Beispiel 14

Ein Gemisch von 4,05 g 3-[4-(4-p-Methoxyphenyl-1,2,3,6-tetrahydro-1-pyridyl)-butyl]-5-nitro-indol, 3,5 g Pyridinhydrochlorid und 80 ml Pyridin wird 3 Std. gekocht. Man kühlt ab, dampft ein, arbeitet wie üblich auf und erhält 3-[4-(4-p-Hydroxyphenyl-1,2,3,6-tetrahydro-1-pyridyl)-butyl]-5-nitro-indol.

Beispiel 15

Eine Suspension von 3,75 g 3-[4-(4-Phenyl-1,2,3,6-tetrahydro-1-pyridyl)-butyl]-5-nitroindol in 45 ml konzentrierter Salzsäure und 30 ml Ethanol wird unter Rühren mit 9,3 g $SnCl_2$ versetzt und dann 0,5 Std. gekocht. Man gießt auf Eis, arbeitet wie üblich auf und erhält 3-[4-(4-Phenyl-1,2,3,6-tetrahydro-1-pyridyl)-butyl]-5-amino-indol, F. 113°.

Beispiel 16

Ein Gemisch von 2 g 3-[4-(4-Phenyl-1,2,3,6-tetrahydro-1-pyridyl)-2-thiabutyl]-5-amino-indol, 2 ml Acetylchlorid und 15 ml Triethylamin wird 16 Std. bei 20° gerührt. Nach üblicher Aufarbeitung erhält man 3-[4-(4-Phenyl-1,2,3,6-tetrahydro-1-pyridyl)-2-thiabutyl]-5-acetamido-indol, F. 172-173°.

Analog erhält man durch Acylierung:

3-[4-(4-Phenyl-1,2,3,6-tetrahydro-1-pyridyl)-butyl]-5-acetamido-indol
3-[4-(4-Phenyl-1,2,3,6-tetrahydro-1-pyridyl)-butyl]-5-benzamido-indol
3-[4-(4-Phenyl-1,2,3,6-tetrahydro-1-pyridyl)-butyl]-5-methylsulfonamido-indol
3-[4-(4-Phenyl-1,2,3,6-tetrahydro-1-pyridyl)-2-thiabutyl]-5-benzamido-indol
3-[4-(4-Phenyl-1,2,3,6-tetrahydro-1-pyridyl)-2-thiabutyl]-5-methylsulfonamido-indol, F. 181-183°.

Beispiel 17

Ein Gemisch von 4,17 g 3-[4-(4-Phenyl-1,2,3,6-tetrahydro-1-pyridyl)-butyl]-indol-5-carbonsäure-N-(2-hydroxyethyl)amid, 1,41 g Benzoylchlorid und 50 ml Triethylamin wird 2 Std. bei 20° gerührt. Nach Eindampfen und üblicher Aufarbeitung erhält man 3-[4-(4-Phenyl-1,2,3,6-tetrahydro-1-pyridyl)-butyl]-indol-5-carbonsäure-N-(2-benzoyloxyethyl)-amid.

Beispiel 18

Ein Gemisch von 4,17 g 3-[4-(4-Phenyl-1,2,3,6-tetrahydro-1-pyridyl)-butyl]-indol-5-carbonsäure-N-(2-hydroxyethyl)-amid, 0,65 g Methylisocyanat und 40 ml Pyridin wird 16 Std. bei 20° gerührt. Nach Eindampfen und üblicher Aufarbeitung erhält man 3-[4-(4-Phenyl-1,2,3,6-tetrahydro-1-pyridyl)-butyl]-indol-5-carbonsäure-N-(2-N-methyl-carbamoyloxyethyl)-amid, F. 117-118°.

Beispiel 19

Man kocht 4,32 g 3-[4-(4-Phenyl-1,2,3,6-tetrahydro-1-pyridyl)-butyl]-5-ethoxycarbonylmethoxy-indol 2 Std. mit 20 ml Wasser und 100 ml 2n ethanolischer KOH, arbeitet wie üblich auf und erhält 3-[4-(4-Phenyl-1,2,3,6-tetrahydro-1-pyridyl)-butyl]-indol-5-oxyessigsäure, Hydrochlorid, Zers. ab 175° (sintert bei 132°).

Die nachstehenden Beispiele betreffen pharmazeutische Zübereitungen, die Amine der Formel I oder ihre Säureadditionssalze enthalten:

Beispiel A: Tabletten

Ein Gemisch von 1 kg 3-[4-(4-Phenyl-1,2,3,6-tetrahydro-1-pyridyl)-butyl]-indol-5-carbonsäure-N-(2-hydroxyethyl)-amid, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

Beispiel B: Dragees

Analog Beispiel A werden Tabletten gepreßt, die anschließend in üblicher Weise mit einem Überzug aus Saccharose, Kartoffelstärke, Talk, Tragant und Farbstoff überzogen werden.

Beispiel C: Kapseln

2 kg 3-[4-(4-Phenyl-1,2,3,6-tetrahydro-1-pyridyl)-2-thiabutyl]-5-indolyl-harnstoff werden in üblicher Weise in Hartgelatinekapseln gefüllt, so daß jede Kapsel 20 mg des Wirkstoffs enthält.

Beispiel D: Ampullen

Eine Lösung von 1 kg 3-[4-(4-Phenyl-1,2,3,6-tetrahydro-1-pyridyl)-butyl]-5-indolyl-harnstoff-dihydrat in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

Analog sind Tabletten, Dragees, Kapseln und Ampullen erhältlich, die einen oder mehrere der übrigen Wirkstoffe der Formel I und/oder ihre physiologisch unbedenklichen Säureadditionssalze enthalten.

**Ansprüche**

1. Indolderivate der Formel I

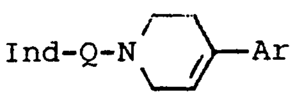

I

worin

Ind einen durch $-O-CH_2-CO-R^1$, $-NHR^2$, $-NO_2$, $-CO-NR^3R^4$ oder $-CSNH_2$ substituierten Indol-3-ylrest,
$R^1$ OH, OA, $NH_2$, NHA, $NA_2$, $NH-CH_2COOA$ oder $NHCH(CH_2OH)COOA$,
$R^2$ H, Ac, $CONH_2$, CONHA, $CONA_2$ oder $SO_2A$,
$R^3$ H, A oder Hydroxyalkyl,
$R^4$ Hydroxyalkyl, AO-alkyl, AcO-alkyl, ANH-CO-O-alkyl, AOOC-alkyl, $H_2NCO$-alkyl, $HSO_3$-alkyl, $A_2N$-alkyl, Ar, Ar-alkyl oder Het-alkyl,
$R^3$ und $R^4$ zusammen auch eine Alkylengruppe mit 3-7 C-Atomen, die durch O oder $NR^5$ unterbrochen und/oder durch $NA_2$, NHAc, COOA, $CONH_2$, Ar oder Het substituiert sein und/oder eine zusätzliche Doppelbindung enthalten kann,
$R^5$ H, A, Ar, Het, Ac, COOA, $CH_2CONH_2$ $CH_2CONHA$, $CH_2CONA_2$ oder $CH_2CONR^6$,
$R^6$ Alkylen mit 3-7 C-Atomen,
Q $-(CH_2)_n-$, $-CH_2-S-CH_2CH_2-$, $-CH_2-SO-CH_2CH_2-$ oder $-CH_2-SO_2-CH_2CH_2-$,
n 2, 3, 4 oder 5,
A Alkyl mit 1-4 C-Atomen,
-alkyl- Alkylen mit 1-4 C-Atomen,
Ar eine unsubstituierte oder eine ein- oder zweifach durch F, Cl, Br, OA und/oder OH oder durch eine Methylendioxygruppe substituierte Phenylgruppe oder eine 2- oder 3-Thienylgruppe,
Ac A-CO- oder Ar-CO- und
Het einen gesättigten oder ungesättigten 5- oder 6-gliedrigen heterocyclischen Rest mit 1-4 N-, O- und/oder S-Atomen, der mit einem Benzolring kondensiert und/oder ein- oder zweifach durch A substituiert sein kann,
bedeuten,
sowie deren Salze,

2.a) 3-[4-(4-Phenyl-1,2,3,6-tetrahydro-1-pyridyl)-butyl]-indol-5-carbonsäure-N-(2-hydroxyethyl)-amid;
b) 3-[4-(4-Phenyl-1,2,3,6-tetrahydro-1-pyridyl)-butyl]-5-indolyl-harnstoff.

3. Verfahren zur Herstellung von Indolderivaten der Formel I nach Anspruch 1 sowie von deren Salzen, dadurch gekennzeichnet, daß man eine Verbindung der Formel II

Ind-Q-$X^1$ II
worin
$X^1$ X oder $NH_2$ und
X Cl, Br, J, OH oder eine reaktionsfähig funktionell abgewandelte OH-Gruppe bedeuten und
Ind und Q die angegebenen Bedeutungen haben,
mit einer Verbindung der Formel III
$X^2$-$CH_2CH_2CAr = CH$-$CH_2X^3$ III
worin
$X^2$ und $X^3$ gleich oder verschieden sein können und, falls $X^1$ = $NH_2$ ist, jeweils X, andernfalls zusammen NH bedeuten und
Ar die angegebene Bedeutung hat,
umsetzt
oder daß man eine sonst der Formel I entsprechende Verbindung, die jedoch an Stelle eines oder mehrerer Wasserstoffatome eine oder mehrere reduzierbare Gruppe(n) und/oder eine oder mehrere zusätzliche C-C- und/oder C-N-Bindung(en) enthält, mit einem reduzierenden Mittel behandelt
oder daß man eine sonst der Formel I entsprechende Verbindung, die jedoch an Stelle eines oder mehrerer Wasserstoffatome eine oder mehrere solvolysierbare Gruppe(n) enthält, mit einem solvolysierenden Mittel behandelt
oder daß man zur Herstellung von Thioethern der Formel I, worin Q -$CH_2$-S-$CH_2CH_2$- bedeutet, eine Verbindung der Formel IV
Ind-$CH_2N(R)_2$ IV
worin
R Alkyl mit 1- 4 C-Atomen, beide Reste R zusammen auch -$(CH_2)_p$- oder -$CH_2CH_2OCH_2CH_2$-und
p 4 oder 5
bedeuten und
Ind die angegebene Bedeutung hat
mit einem Thiol der Formel V

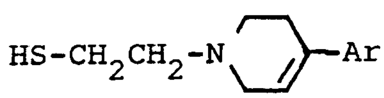 V

worin
Ar die angegebene Bedeutung hat
oder einem seiner reaktionsfähigen Derivate umsetzt
oder daß man eine Verbindung der Formel VI

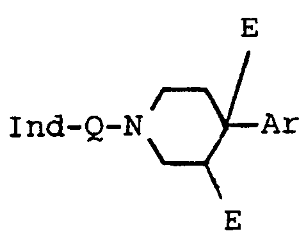 VI

worin
der eine Rest E X, CN oder $NH_2$,
der andere Rest E H bedeutet und
Ind, Q, Ar und X die angegebenen Bedeutungen haben,
mit einem HE-abspaltenden Mittel behandelt
oder daß man zur Herstellung einer Verbindung der Formel I, worin Ind einen durch -O-$CH_2$-CO-$R^1$ substituierten Indol-3-ylrest bedeutet, ein Hydroxyindol, das sonst der Formel I entspricht, aber an Stelle von Ind einen durch eine OH-Gruppe substituierten Indol-3-ylrest enthält, oder eines seiner reaktionsfähigen Derivate mit einer Verbindung der Formel X-$CH_2$-CO-$R^1$ (worin X und $R^1$ die angegebenen Bedeutungen haben) umsetzt
oder daß man zur Herstellung einer Verbindung der Formel I, worin Ind einen durch -CO-$NR^3R^4$ substituier-

ten Indol-3-ylrest bedeutet, eine Indolcarbonsäure, die sonst der Formel I entspricht, aber an Stelle von Ind einen durch eine COOH-Gruppe substituierten Indol-3-ylrest enthält, oder eines ihrer reaktionsfähigen Derivate mit einer Verbindung der Formel $HNR^3R^4$ (worin $R^3$ und $R^4$ die angegebenen Bedeutungen haben) umsetzt

oder daß man zur Herstellung einer Verbindung der Formel I, worin Ind einen durch $-CS-NH_2$ substituierten Indol-3-ylrest bedeutet, ein Cyanindol, das sonst der Formel I entspricht, aber an Stelle von Ind einen durch eine CN-Gruppe substituierten Indol-3-ylrest enthält, mit $H_2S$ oder einem $H_2S$-abgebenden Mittel umsetzt

und/oder daß man gegebenenfalls in einer Verbindung der Formel I eine Thioethergruppe zu einer SO-Gruppe oder $SO_2$-Gruppe oder eine SO-Gruppe zu einer $SO_2$-Gruppe oxydiert und/oder eine OA-Gruppe unter Bildung einer OH-Gruppe spaltet und/oder eine Gruppe Ind in eine andere Gruppe Ind umwandelt und/oder daß man eine erhaltene Base der Formel I durch Behandeln mit einer Säure oder Base in eines ihrer Salze umwandelt.

4. Verfahren zur Herstellung pharmazeutischer Zübereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger-oder Hilfsstoff und gegebenenfalls in Kombination mit einem oder mehreren weiteren Wirkstoffen in eine geeignete Dosierungsform bringt.

5. Pharmazeutische Zübereitung, gekennzeichnet durch einen Gehalt an mindestens einer Verbindung der allgemeinen Formel I und/oder einem ihrer physiologisch unbedenklichen Salze.

6. Verwendung von Verbindungen der Formel I nach Patentanspruch 1 oder von deren physiologisch unbedenklichen Salzen zur Herstellung eines Arzneimittels.

7. Verwendung von Verbindungen der Formel I nach Patentanspruch 1 oder von deren physiologisch unbedenklichen Salzen bei der Bekämpfung von Krankheiten.

Europäisches Patentamt

**EUROPÄISCHER TEILRECHERCHENBERICHT,**
der nach Regel 45 des Europäischen Patent-übereinkommens für das weitere Verfahren als europäischer Recherchenbericht gilt

Nummer der Anmeldung: EP 90103842.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | DE - A1 - 3 236 243 (MERCK) * Anspruch 1 * -- | 1 | C 07 D 401/12<br>C 07 D 401/06<br>C 07 D 409/14<br>A 61 K 31/44 |
| A | DE - A1 - 3 308 668 (MERCK) * Ansprüche 1,4,5 * -- | 1,4,5 | |
| A | DE - A1 - 3 342 632 (MERCK) * Ansprüche 1,4,5; Seite 31, Zeilen 4-19 * -- | 1,4,5 | |
| A | DE - A1 - 2 827 874 (MARCK) * Ansprüche 1,4,5 * -- | 1,4,5 | |
| A | DE - A1 - 3 419 935 (MERCK) * Anspruch 1 * -- | 1 | RECHERCHIERTE SACHGEBIETE (Int. Cl.5) |
| A | DE - A1 - 3 604 949 (MERCK) * Ansprüche 1,4,5; Zusammen- | 1,4,5 | C 07 D 401/00<br>C 07 D 409/00 |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-6
Unvollständig recherchierte Patentansprüche: -
Nicht recherchierte Patentansprüche: 7
Grund für die Beschränkung der Recherche:

Artikel 52(4) EPÜ; Verfahren zur therapeutischen Behandlung des menschlichen oder tierischen Körpers)

| Recherchenort | Abschlußdatum der Recherche | Prüfer |
|---|---|---|
| WIEN | 02-05-1990 | HAMMER |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

| EINSCHLÄGIGE DOKUMENTE | | | KLASSIFIKATION DER ANMELDUNG (Int. Cl.4)5 |
|---|---|---|---|
| Kategorie | Kennzeichnung des Dokuments mit Angabe, soweit erforderlich, der maßgeblichen Teile | betrifft Anspruch | |
| | fassung * <br> -- | | |
| A | CHEMICAL ABSTRACTS, Band 109, Nr. 13, 26. September 1988, Columbus, Ohio, USA BOETTCHER, HENNING et al. "Synthesis of 3-(4-(1,2,3,6--tetrahydro-4-phenyl-1--pyridyl)butyl)-5-indolecarboxylic acid, an antihypertensive agent with a novel mode of action" Seite 600, Spalte 1, Zusammenfassung Nr. 110 201t & Liebigs Ann. Chem. 1988, (8), 749-52 <br> ---- | 1 | |
| | | | RECHERCHIERTE SACHGEBIETE (Int. Cl.4)5 |